# FASCICULE DE BREVET EUROPEEN

(11) **EP 0 534 810 B1**
(45) Date de publication et mention de la délivrance du brevet: **15.11.1995**
(21) Numéro de dépôt: 92402338.5
(22) Date de dépôt: 25.08.1992
(51) Int. Cl.: A61K 7/04

(54) **Composition pour le traitement des ongles contenant un aminoacide soufre**
Mittel zur Behandlung von Nägeln enthaltend eine sulfurierte Aminosäure
Nailcare composition containing an amino acid comprising sulfur

(30) Priorité: 26.09.1991 FR 9111842
(43) Date de publication de la demande: 31.03.1993
(73) Titulaire: L'OREAL, 75008 Paris (FR)
(72) Inventeur: Agnus, Dominique, F-94210 La Varenne Saint-Hilaire (FR); Ser, Jean-Claude, F-94550 Chevilly Larue (FR)
(74) Mandataire: Peuscet, Jacques

(56) Documents cités:
- FR-M- 3 111
- STN SERVEUR DE BASES DE DONNEES, KARLSRUHE DE, FICHIER CHEMICAL ABSTRACTS, vol.114, no. 6, résumé no. 47427g, Columbus, Ohio, US; & PL-A-147 588 (INSTYTUT MECHANIKI PRECYZYJNEJ) 31-10-1989
- WORLD PATENT INDEX, semaine 7841, AN=78-73629A, Derwent Publications Ltd,Londres, GB; & JP-A-53 101 542 (MATSUSHITA ELEC. WORKS) 05-09-1978
- VIDAL, Dictonnaire de la chimie et de ses applications, 3e édition, Technique et documentation, Paris (FR)

## Description

La présente invention concerne une composition pour le traitement des ongles contenant un aminoacide soufré.

Il est connu que les aminoacides soufrés et, notamment, la S-carboxyméthylcystéine ont un rôle important dans le processus de kératinisation lors de la formation de l'ongle, et sont, par conséquent, susceptibles d'être utilisés pour le traitement des ongles abîmés et fragilisés pour les rendre plus durs et plus solides et améliorer leur aspect.

Dans le brevet spécial médicament français 3111M on décrit le traitement des ongles à l'aide d'un médicament, en particulier d'une crème, contenant de la S-carboxyméthylcystéine.

Dans FR-A 1603 799 on décrit l'utilisation pour l'entretien des cheveux et des ongles, d'acylméthionines dans lesquelles le groupe acyle est en C₆ -C₂₂.

Dans FR-A 2503151 est décrite l'utilisation pour le traitement des ongles de dérivés de la cystine ou de la cystéine substitués par un groupe butyryle sur la fonction amine.

Dans la demande de brevet japonais 53 101 542, on décrit une composition pour le traitement des cheveux contenant comme agent réducteur la cystéine ou un dérivé de cystéine et un tampon constitué par du phosphate monopotassique et du tétraborate de sodium.

Par ailleurs il est connu, de façon générale, que le tétraborate de sodium peut être utilisé comme base pour la neutralisation d'acides.

La présente invention a pour objet une composition pour le traitement des ongles contenant un aminoacide soufré ou un dérivé d'aminoacide soufré caractérisée par le fait qu'elle est susceptible d'être préparée en introduisant dans un milieu aqueux ou hydroalcoolique à la fois un aminoacide soufré et un tétraborate de sodium afin d'obtenir une solution tamponnée ayant un pH voisin de 7,5.

On a constaté que dans cette composition, le tétraborate de sodium a deux fonctions :
1) il neutralise l'aminoacide soufré en formant du sel de sodium ; lorsque l'aminoacide soufré n'est pas soluble à froid dans l'eau, comme c'est le cas pour la S-carboxyméthylcystéine, cette neutralisation permet de le solubiliser ;
2) il a un effet tampon, ce qui permet de maintenir la solution obtenue à un pH voisin de 7,5, pH qui est suffisamment élevé pour permettre un ramollissement et un gonflement des cuticules, mais suffisamment faible pour que la peau et la chair autour de l'ongle ne soient pas attaquées.

Par conséquent, la composition selon l'invention permet à la fois de traiter les ongles pour éviter qu'ils ne deviennent fragiles ou mous et de traiter les cuticules pour faciliter leur élimination.

Le milieu dans lequel sont dissous, selon l'invention, l'aminoacide soufré et le tétraborate de sodium est un milieu aqueux ou hydroalcoolique. Dans ce dernier cas, l'alcool utilisé est de préférence, un monoalcool en C₁ - C₃. La proportion d'alcool est, de préférence, comprise entre 0 et 35% en poids par rapport au milieu aqueux.

L'aminoacide soufré est, de préférence choisi dans le groupe formé par la méthionine, la cystéine, la S-carboxyméthylcystéine et leurs dérivés. Dans ce groupe la S-carboxyméthylcystéine est préférée.

De préférence, l'aminoacide soufré est introduit dans la composition dans une proportion comprise entre 0,5 et 5% en poids par rapport au poids total de la composition et le tétraborate de sodium est introduit dans la composition dans une proportion comprise entre 0,5 et 5% en poids par rapport au poids total de la composition, le rapport en poids aminoacide soufré/ tétraborate de sodium étant compris entre 2 et 0,5, de préférence entre 1 et 0,5.

Les compositions selon l'invention peuvent contenir des actifs cosmétiques et/ou pharmaceutiques hydrosolubles. Parmi ces actifs on peut citer :
- les alcools polyhydriques tels que la glycérine, l'éthylène glycol, le propane diol-1,2, et l'érythritol,
- les acides aminés non soufrés tels que la proline, l'arginine, la lysine, l'histidine,
   l'hydroxyproline et leurs dérivés,
- les agents antifongiques, antiseptiques et antimicrobiens, tels que le chlorure de benzalkonium ou de benzéthonium, le diiséthionate d'hexamédine, le digluconate de chloréxidine et les dérivés de pyridinethione sous forme acide, plus particulièrement, ceux vendus sous la marque "OMADINE" par la société OLIN CHEMICALS et
- les vitamines, par exemple le panthénol et la vitamine H.

On peut également introduire un adjuvant cosmétiquement et/ou pharmaceutiquement acceptable pris dans le groupe formé par :
- les agents de texture ou agents épaississants tels que les hydroxyalkylcelluloses, les homopolymères ou copolymères acryliques, les gommes de xanthane, la gomme adragante, la gomme arabique et les copolymères vinylpyrrolidone/vinylacétate ;
- les agents conservateurs tels que le parahydroxybenzoate de méthyle ou le parahydroxybenzoate de butyle ;
- des colorants tels que FD & C yellow n°5, FD & C red n°4, FD & C blue n°1, D & C Green n°5, FD & C green n°3, D & C red n°33, FD & C red n°40, D & C yellow n°10, D & C violet n°2,
- des parfums et
- des filtres UV.

Pour traiter les ongles et les cuticules l'utilisateur peut soit tremper l'extrémité des doigts dans un récipient contenant la composition selon l'invention, soit déposer la composition sur les ongles et les cuticules à l'aide d'un pinceau ou de tout autre applicateur approprié.

Les exemples donnés ci-après à titre purement illustratif et non limitatif permettront de mieux comprendre l'invention.

### Exemple 1

On prépare en introduisant dans de l'eau les différents composants, une composition ayant la formulation suivante en poids.

| | |
|---|---|
| S-Carboxyméthylcystéine | 1 g |
| Tétraborate de sodium | 2 g |
| Ethanol | 20 g |
| Glycérine | 5 g |
| Parahydroxybenzoate de méthyle | 0,5 g |
| Polymère acrylique | 0,5 g |
| Eau | qsp 100 g |

On a constaté qu'une application de la composition obtenue par trempage de l'extrémité des doigts pendant 5 minutes permet d'éliminer facilement les cuticules et que si cette application est renouvelée de façon bi-hebdomadaire, pendant deux mois, l'aspect des ongles est très nettement amélioré et l'ongle est plus dur et moins fragile.

### Exemple 2

On a préparé comme dans l'exemple 1 une composition ayant la formulation suivante en poids :

| | |
|---|---|
| S-Carboxyméthylcystéine | 5 g |
| Tétraborate de sodium | 5 g |
| Isopropanol | 10 g |
| Polyvinylpyrrolidone | 0,3 g |
| Gomme de xanthane | 0,5 g |
| Parahydroxybenzoate de méthyle | 0,5 g |
| Pyrrolidone carboxylate d'arginine | 1 g |
| Eau | qsp 100 g |

On a effectué les mêmes constatations que pour la composition de l'exemple 1.

### Exemple 3

On a préparé comme dans l'exemple 1 une composition ayant la formulation suivante en poids :

| | |
|---|---|
| s-Carboxyméthylcystéine | 2 g |
| Tétraborate de Sodium | 4 g |
| Ethanol | 30 g |
| Hydroxyalkylcellulose | 0,6 g |
| Copolymère vinylpyrrolidone/Vinyl acétate | 0,3 g |
| D - panthénol | 3 g |
| D & C Red N°33 | 0,0004 g |
| Eau | qsp 100 g |

On a effectué les mêmes constatations que pour la composition de l'exemple 1.

## Revendications

1. Composition pour le traitement des ongles contenant un aminoacide soufré ou un dérivé d'aminoacide soufré, caractérisée par le fait qu'elle est susceptible d'être préparée en introduisant dans un milieu aqueux ou hydroalcoolique à la fois un aminoacide soufré et un tétraborate de sodium afin d'obtenir une solution tamponnée ayant un pH voisin de 7,5.

2. Composition selon la revendication 1, caractérisée par le fait que le milieu hydroalcoolique contient un monoalcool en C₁ -C₃ .

3. Composition selon l'une des revendication 1 ou 2, caractérisée par le fait que la proportion d'alcool est comprise entre 0 et 35% en poids par rapport au milieu aqueux.

4. Composition selon l'une des revendications 1 à 3, caractérisée par le fait que l'aminoacide soufré est choisi dans le groupe formé par la méthionine, la cystéine et la S-carboxyméthylcystéine.

5. Composition selon l'une des revendications 1 à 4, caractérisée par le fait que l'aminoacide soufré est introduit dans une proportion comprise entre 0,5 et 5% en poids par rapport au poids total de la composition et que le tétraborate de sodium est introduit dans une proportion comprise entre 0,5 et 5% en poids par rapport au poids total de la composition, le rapport en poids aminoacide soufré/tétraborate de sodium étant compris entre 2 et 0,5.

6. Composition selon la revendication 5, caractérisée par le fait que le rapport en poids aminoacide soufré/tétraborate de sodium est comprise entre 1 et 0,5.

7. Composition selon l'une des revendications 1 à 6, caractérisée par le fait qu'elle contient des actifs cosmétiques et/ou pharmaceutiques hydrosolubles.

8. Composition selon la revendication 7, caractérisée par le fait que les actifs hydrosolubles sont choisis dans le groupe formé par les alcools polyhydriques, les acides aminés non soufrés, les agents antifongiques, antiseptiques et antimicrobiens et les vitamines.

9. Composition selon l'une des revendications 1 à 8, caractérisée par le fait qu'elle contient un adjuvant cosmétiquement et/ou pharmaceutiquement acceptable.

10. Composition selon la revendication 9, caractérisée par le fait que l'adjuvant est choisi dans le groupe formé par les agents de texture, les colorants, les parfums et les filtres UV.

## Claims

1. Composition for treating the nails, comprising a sulphur-containing amino acid or a sulphur-containing amino acid derivative, characterized in that it is capable of being prepared by introducing into an aqueous or aqueous-alcoholic medium both a sulphur-containing amino acid and a sodium tetraborate so as to obtain a buffered solution having a pH close to 7.5.

2. Composition according to Claim 1, characterized in that the aqueous-alcoholic medium contains a C₁-C₃ monoalcohol.

3. Composition according to either of Claims 1 and 2, characterized in that the proportion of alcohol is between 0 and 35 % by weight relative to the aqueous medium.

4. Composition according to one of Claims 1 to 3, characterized in that the sulphur-containing amino acid is chosen from the group consisting of methionine, cysteine and S-carboxymethylcysteine.

5. Composition according to one of Claims 1 to 4, characterized in that the sulphur-containing amino acid is introduced in a proportion of between 0.5 and 5 % by weight relative to the total weight of the composition and in that the sodium tetraborate is introduced in a proportion of between 0.5 and 5 % by weight relative to the total weight of the composition, the sulphur-containing amino acid/sodium tetraborate weight ratio being between 2 and 0.5.

6. Composition according to Claim 5, characterized in that the sulphur-containing amino acid/sodium tetraborate ratio is between 1 and 0.5.

7. Composition according to one of Claims 1 to 6, characterized in that it contains water-soluble cosmetic and/or pharmaceutical active agents.

8. Composition according to Claim 7, characterized in that the water-soluble active agents are chosen from the group consisting of polyhydric alcohols, non-sulphur-containing amino acids, antifungal, antiseptic and antimicrobial agents and vitamins.

9. Composition according to one of Claims 1 to 8, characterized in that it contains a cosmetically and/or pharmaceutically acceptable adjuvant.

10. Composition according to Claim 9, characterized in that the adjuvant is chosen from the group consisting of texturing agents, colourants, perfumes and UV-screening agents.

## Patentansprüche

1. Mittel zur Behandlung von Nägeln, das eine sulfurierte Aminosäure oder ein Derivat einer sulfurierten Aminosäure enthält, dadurch gekennzeichnet, daS es herstellbar ist, indem man in ein wäßriges oder wäßrigalkoholisches Milieu eine sulfurierte Aminosäure und ein Natriumtetraborat gemeinsam einbringt, um eine gepufferte Lösung mit einem pH von etwa 7,5 zu erhalten.

2. Mittel nach Anspruch 1, dadurch gekennzeichnet, daß das wäßrigalkoholische Milieu einen Monoalkohol mit 1 bis 3 Kohlenstoffatomen enthält.

3. Mittel nach einem der Ansprüche 1 oder 2, dadurch gekennzeichnet, daß der Alkoholanteil 0 bis 35 Gew.-%, bezogen auf das wäßrige Milieu, beträgt.

4. Mittel nach einem der Ansprüche 1 bis 3, dadurch gekennzeichnet, daß die sulfurierte Aminosäure ausgewählt ist unter Methionin, Cystein und S-Carboxymethylcystein.

5. Mittel nach einem der Ansprüche 1 bis 4, dadurch gekennzeichnet, daß die sulfurierte Aminosäure in einem Anteil von 0,5 bis 5 Gew.-%, bezogen auf das Gesamtgewicht des Mittels, hinzugegeben wird, und das Natriumtetraborat in einem Anteil von 0,5 bis 5 Gew.-%, bezogen auf das Gesamtgewicht des Mittels, hinzugegeben wird, so daß das Gewichtsverhältnis sulfurierte Aminosäure/Natriumtetraborat 2 bis 0,5 beträgt.

6. Mittel nach Anspruch 5, dadurch gekennzeichnet, daß das Gewichtsverhältnis sulfurierte Aminosäure/Natriumtetraborat 1 bis 0,5 beträgt.

7. Mittel nach einem der Ansprüche 1 bis 6, dadurch gekennzeichnet, daß es kosmetische und/oder pharmazeutische, wasserlösliche Wirkstoffe enthält.

8. Mittel nach Anspruch 7, dadurch gekennzeichnet, daß die wasserlöslichen Wirkstoffe ausgewählt sind unter Polyhydroxyalkoholen, nicht sulfurierten Aminosäuren, antifungischen, antiseptischen und antimikrobiellen Mitteln und Vitaminen.

9. Mittel nach einem der Ansprüche 1 bis 8, dadurch gekennzeichnet, daß es kosmetische und/oder pharmazeutisch akzeptable Zusatzstoffe enthält.

10. Mittel nach Anspruch 9, dadurch gekennzeichnet, daß der Zusatzstoff ausgewählt ist unter Texturierungsmitteln, Farbstoffen, Parfüms und UV-Filtern.
